(19) Europäisches Patentamt / European Patent Office / Office européen des brevets

(11) **EP 4 684 731 A1**

(12) **EUROPEAN PATENT APPLICATION**

(43) Date of publication:
**28.01.2026  Bulletin 2026/05**

(21) Application number: **24315354.1**

(22) Date of filing: **23.07.2024**

(51) International Patent Classification (IPC):
**A61B 5/375** (2021.01)   **A61M 21/02** (2006.01)
**G06F 3/01** (2006.01)

(52) Cooperative Patent Classification (CPC):
(C-Sets available)
**A61B 5/375; A61B 5/7278; A61M 21/00;**
**A61M 21/02; G06F 3/015;** A61B 5/38;
A61M 2021/0016; A61M 2021/0022;
A61M 2021/0027; A61M 2021/005;
A61M 2021/0083; A61M 2205/50; A61M 2230/04;
A61M 2230/10; A61M 2230/40          (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicants:
• **Centre National de la Recherche Scientifique**
  **75016 Paris (FR)**

• **Université Paris-Saclay**
  **91190 Gif-sur-Yvette (FR)**

(72) Inventor: **DESTEXHE, Alain**
  **91190 Gif-sur-Yvette (FR)**

(74) Representative: **Hautier IP**
  **20, rue de la Liberté**
  **06000 Nice (FR)**

(54)  **PHYSIO-SENSORY TRANSDUCTION METHOD AND DEVICE**

(57)     The invention relates to a physio-sensory transduction method comprising: an acquisition (E1) of a physiological signal (10) of an organism comprising a raw sequence ($S_r$) of at least one raw pattern (100), and a generation (E2) of a sensory signal (30) associated with the raw sequence ($S_r$). The generation (E2) of the sensory signal (30) comprises a transformation (E21) of the raw sequence ($S_r$) into a modulated sequence ($S_m$), said modulated sequence ($S_m$) comprising at least one modulated pattern (101), wherein each modulated pattern (101) is obtained by fitting the raw pattern (100) with a sum of at least one Gaussian function ($f_G$), said sum of at least one Gaussian function forming an envelope to each raw pattern.

FIG.1

EP 4 684 731 A1

(52) Cooperative Patent Classification (CPC): (Cont.)

C-Sets
A61M 2230/04, A61M 2230/005;
A61M 2230/10, A61M 2230/005;
A61M 2230/40, A61M 2230/005

**Description**

TECHNICAL FIELD

The present invention relates to the fields of signal processing and sensory stimulation.

More specifically, the present invention relates to a method and a device for the transduction of a physiological signal into a sensory signal. Non-limitatively, the physiological signal can be representative of a brain activity of a human or animal being, and the sensory signal can be an acoustic signal.

PRIOR ART

Methods and devices intended to stimulate an individual's brain waves based on measurements of brain activity of the individual have garnered significant interest in scientific and technological realms. The detection and the quantification of brain activities are common knowledge in the state of the art. However, the translation of physiological signals into actionable sensory feedback remains a key challenge.

For example, in the patent publication FR 3065366 B1, the transcription of a physiological signal into a sensory signal, is done using an ADSR language which stands for Attack, Decay, Sustain, Release. These are the four stages of an ADSR envelope, that is used to control different parameters in a synth, including for instance amplitude, duration, starting time, ending time, *etc.* The approach used in the above-mentioned document, is based on parameterizing patterns detected in the physiological signal, and on generating sensory signals determined as a function of this parameterization. These parameters, defined in the ADSR language, are then input into a synthesizer to generate the sensory signal.

A purpose of the present invention is to provide an alternative method to transcribe a physiological signal into a sensory signal. Another purpose of the present invention, is to improve, compared with the state of the prior art, the transcription of a physiological signal - in particular representative of a brain activity - in the form of a sensory signal - acoustic or other - perceptible to a human user.

SUMMARY

To at least one of these ends, it is provided a physio-sensory transduction method comprising:

-    an acquisition of a physiological signal of an organism, said physiological signal comprising a raw sequence of at least one raw pattern, and
-    a generation of a sensory signal associated with the raw sequence,

the generation of the sensory signal comprising a transformation of the raw sequence into a modulated sequence, said modulated sequence comprising at least one modulated pattern, wherein each modulated pattern is obtained by fitting the raw pattern with a sum of at least one Gaussian function, said sum of at least one Gaussian function forming an envelope to each raw pattern.

The method described hereabove, allows by using a sum of Gaussian functions, to transduce accurately and continuously the physiological signal into a sensory signal. In fact, the sum of Gaussian functions fits seamlessly the patterns in the physiological signal in a personalized way, which enables to reproduce the fine structure of the physiological signal. Besides the accurate physio-sensory transduction, this method is not limited to the ADSR language, which makes it more flexible in terms of signal processing. In fact, the present method does not require any parametrization of the patterns, which simplifies the processing of the physiological signal.

Moreover, the generation of the sensory signal does not necessarily require the use of synthesizers. This allows, in the case of physio-acoustic transduction for instance, the generation of a wide range of sounds, such as voices or natural sounds. Such a generalization of sounds is not possible with the ADSR method because it is limited to the sounds of a synthesizer. The present method can therefore be fully automated, given that its steps are numerical, from the acquisition of the physiological signal to the generation of the sensory signal. This simplifies the application of the method.

Another aspect of the invention relates to a physio-sensory transduction device comprising:

-    an acquisition means configured to acquire a physiological signal from an organism, said physiological signal comprising a raw sequence of at least one raw pattern, and
-    a generation system configured to generate a sensory signal associated with the raw sequence,

the generation system being further configured to transform the raw sequence into a modulated sequence, said modulated sequence comprising at least one modulated pattern, wherein each modulated pattern is obtained by fitting the raw pattern with a sum of at least one Gaussian function. Such a device makes it possible to implement the method described above.

BRIEF DESCRIPTION OF THE FIGURES

The foregoing and other aspects of the embodiments of this invention are made more evident in the following Detailed Description, when read in conjunction with the attached Drawing Figures, wherein:

Figure 1 illustrates a diagram showing steps of the method according to an embodiment of the present invention.

Figure 2 illustrates an example of a physiological signal acquired from an organism.

Figure 3 illustrates a part of the physiological signal of Figure 1, representing a raw pattern and an associated modulated pattern.

Figure 4A illustrates a modulated sequence according to an embodiment of the present invention.

Figure 4B illustrates a modulated sequence according to another embodiment of the present invention.

Figure 5 illustrates an example of a slow periodic function.

Figure 6 illustrates an example of a constant function.

The figures are given as example and are not restrictive to the invention. They are guideline schematic representations designed to facilitate the understanding of the invention and they are not necessarily at the scale of practical implementations.

DETAILED DESCRIPTION

According to an embodiment, the modulated sequence comprises at least two successive modulated patterns, and the transformation of the raw sequence is configured in a way that the sums of at least one Gaussian function, associated with the at least two successive modulated patterns, overlap. This allows obtaining a modulated sequence that is continuous without sudden changes. A calming and natural auditory experience, akin to a melody, can thus be generated.

According to an embodiment, the generation of the sensory signal further comprises a first sub-generation of a periodic function that is added to the modulated sequence, said periodic function having a periodicity comprised between 10 seconds and 20 seconds.

The periodic function is labeled as "slow", relatively to the modulated sequence, given that its periodicity is preferably slower than that of an average periodicity of the modulated sequence. In other words, as the slow periodic function completes one cycle, 5 to 80 patterns can occur in the modulated sequence.

The slow periodic function represents a global modulation of the sensory signal.

In an embodiment, the slow periodic function is independent from the raw sequence. This independence from the raw physiological signal, distinguishes the present method from conventional ones. In fact, in the state of the art, periodic functions are generated in accordance with the physiological signal. The independence of the periodic function from the physiological signal allows the generation of a sensory signal whose amplitude is different than the amplitude of the physiological signal, which further simplifies the processing of the signals. Moreover, the slow periodic function, allows the generation of a more soothing sound.

According to an example, the periodicity of the periodic function is comprised between 10 seconds and 20 seconds.

Such a slow periodicity is slower than the average breathing period of a human being that lies in the range of 3 to 5 seconds approximately. This helps generating a sound that is more relaxing.

According to an example, the periodic function has a maximal amplitude $A_s$ that represents a first fraction of a maximal amplitude $A_{30}$ of the generated sensory signal, said first fraction being comprised between 20% and 40%.

According to an embodiment, the generation of the sensory signal further comprises a second sub-generation of a constant function that is added to the modulated sequence, said constant function having a constant amplitude $A_c$ greater than zero.

Adding a constant function to the modulated envelope, allows the generation of a sustained sensory signal, or a sustained sound in the case of a physio-acoustic transduction. In fact, the constant non-zero amplitude $A_c$ of the constant function, helps avoiding interruptions in the generated sound, or moments of silence between two successive patterns. This allows to obtain a sound that is more pleasant to listen to.

According to an example, the constant amplitude $A_c$ of the constant function represents a second fraction of a maximal amplitude $A_{30}$ of the generated sensory signal, said second fraction being comprised between 20% and 40%.

According to an example, the modulated sequence has a maximal amplitude $A_G$ that represents a third fraction of a maximal amplitude $A_{30}$ of the generated sensory signal, said third fraction being comprised between 20% and 40%.

The generated sensory signal can thus comprise three different components, namely, a first component represented by the sums of Gaussian functions, a second component represented by the slow periodic function and a third component represented by the constant function. The first component follows closely the shape of the physiological signal but does not produce the maximal amplitude of the sensory signal. The contribution of the second and third component, that are independent from the physiological signal, to the maximal amplitude of the sensory signal, help enhancing the sensory signal and the auditory experience of the organism.

According to an embodiment, each sum of at least one Gaussian function comprises a single Gaussian function. Using a single Gaussian function to fit one raw pattern, produces a symmetrical curve that fits a large part of the raw pattern.

According to an embodiment, each sum of at least one Gaussian function comprises at least two Gaussian functions. The use of a sum of more than two Gaussian functions to fit one raw pattern, produces a curve that fits closely with the raw pattern, without being necessarily symmetrical. This embodiment allows to generate a modulated sequence that faithfully reflects the raw sequence.

According to an example, the generation of the sensory signal is carried out using a sequence of pre-recorded signals modulated by at least the modulated sequence. The pre-recorded signals can be for example natural sounds including water flowing sound, waves sound, rain falling sound, whales sound, *etc.* These sounds, when modulated to the physiological signal, can be used to help increasing the quality of sleeping of the individual.

According to an example, the generation of the sensory signal is carried out in a deferred manner with respect to the acquisition of the physiological signal. In other words, the generation of the sensory signal(s) can be preceded by a recording of computer data or of a digital or electronic or electric or analogue signal (for example a sound file) allowing the subsequent generation of the sensory signal(s).

According to an example, the generation of the sensory signal is carried out in real time with respect to the acquisition of the physiological signal. By the expression "real time" is meant that the processing of the physiological signal (or the different steps concerned) is carried out in such a way that the modulation of the sensory signal generated is representative of the physiological signal acquired at the same moment, plus or minus the time of implementation of the processing steps.

According to an example:

- the organism is a human being or an animal,
- the physiological signal is representative of a brain activity of said human being or said animal, and
- the sensory signal is an acoustic signal.

The physiological signal is preferably representative of a brain activity of the organism which can, for example, be measured by electroencephalography, or any other technique for recording brain activity.

Alternatively, the physiological signal is representative of a cardiac or respiratory or ocular or muscular activity of the organism.

The sensory signal is preferably an acoustic signal (acoustic wave having as signal parameter, for example, a duration and/or a frequency or frequencies and/or an intensity of this frequency or these frequencies) audible to a human being.

Alternatively, the sensory signal can be:

- a visual signal (image or light having as signal parameter, for example, a duration and/or an intensity or amplitude and/or a frequency or wavelength (of each pixel in the case of an image)),
- tactile (mechanical pressure having as signal parameter, for example, a duration and/or a force and/or an amplitude of movement and/or a vibration frequency),
- olfactory (odour having as signal parameter, for example, a duration and/or an intensity or amplitude and/or a scent), or
- gustatory (taste having as signal parameter, for example, a duration and/or an intensity or amplitude and/or a flavour).

In an embodiment, the pattern(s) in the physiological signal can be representative of a state of deep sleep. The pattern(s) can in this case consist of waves with a frequency comprised between 0.3 Hz and 5 Hz called delta waves.

In a preferred embodiment, the device can be arranged and/or programmed in such a way that:

- the organism can be a human being or an animal,
- the physiological signal is representative of a brain activity of said human being or said animal, and
- the sensory signal is an acoustic signal.

The steps of the method are broadly understood as the implementation of a portion of the methods and may potentially be carried out in multiple sub-steps. Several embodiments of the invention involving successive steps of the method are described below. Unless explicitly stated, the adjective "successive" does not necessarily imply, although it is generally preferred, that the steps immediately follow one another, with intermediate steps potentially separating them.

Furthermore, the term "step" does not necessarily imply that the actions taken during a step are simultaneous or immediately successive. Certain actions of a first step may, in particular, be followed by actions related to a different step, and other actions of the first step may be resumed later. Thus, the term "step" does not necessarily refer to unitary and inseparable actions in time and in the sequence of method phases.

The terms "substantially", "approximately", "about" mean, when referring to a value, "within 10%" of that value.

The term "envelope" designates a smooth curve that describes the evolution of a signal. It outlines the changes in the signal. The envelope may be a function of time, space, angle, or indeed of any variable.

The term "raw pattern" designates an unprocessed part or feature of a physiological signal in its original form, without any applied modifications, transformations, or fitting. This raw pattern corresponds to a significant part of the physiological signal, that can be differentiated from noise or artefacts in the signal.

The term "raw sequence" designates a continuous succession of raw patterns.

The term "modulated pattern" designates an arrangement of data points that have been calculated numerically to fit or modulate a raw pattern. These data points form a curve that has undergone a controlled variation in one or more of its parameters (e.g. amplitude, frequency, phase, etc.), according to a specific method, in order to fit as closely as possible the raw pattern.

The term "modulated sequence" designates a continuous succession of modulated patterns.

The following detailed description of the invention refers to the accompanying drawings. While the description includes exemplary embodiments, other embodiments are possible, and changes may be made to the embodiments described without departing from the spirit and scope of the invention.

Some advantageous features will be described below. Then some exemplary embodiments and use cases will be further detailed in regard with the drawings.

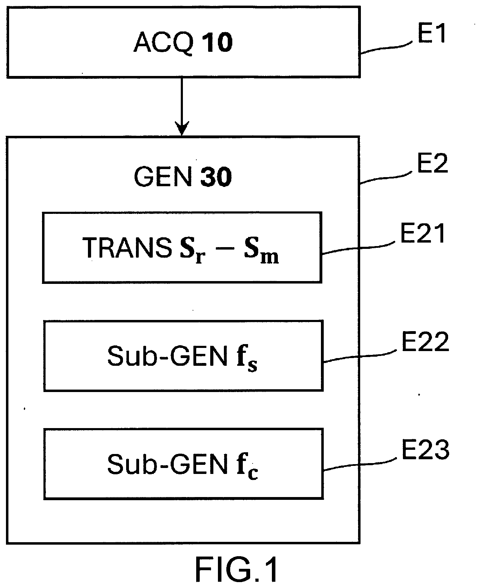

Figure 1 shows a diagram of different steps of the physio-sensory method according to an embodiment of the present invention. The method comprises a step of acquisition E1 of a physiological signal 10 of an organism. In the box E1 of the diagram of Figure 1, which corresponds to the step of acquisition of the physiological signal, "ACQ 10" means "acquisition of the physiological signal 10".

The physiological signal 10 is preferably a temporal signal, *i.e.* a signal which is a function of a time. The physiological signal 10 may have temporal dynamics, i.e. it varies over time.

The physiological signal 10, comprises a raw sequence $S_r$ of at least one raw pattern 100. The term "raw" is used to refer to patterns present in the physiological signal 10 which has just been acquired, these patterns remaining unprocessed or modulated. The raw sequence $S_r$ may comprise one or more unprocessed raw patterns 100 within an elapsed period.

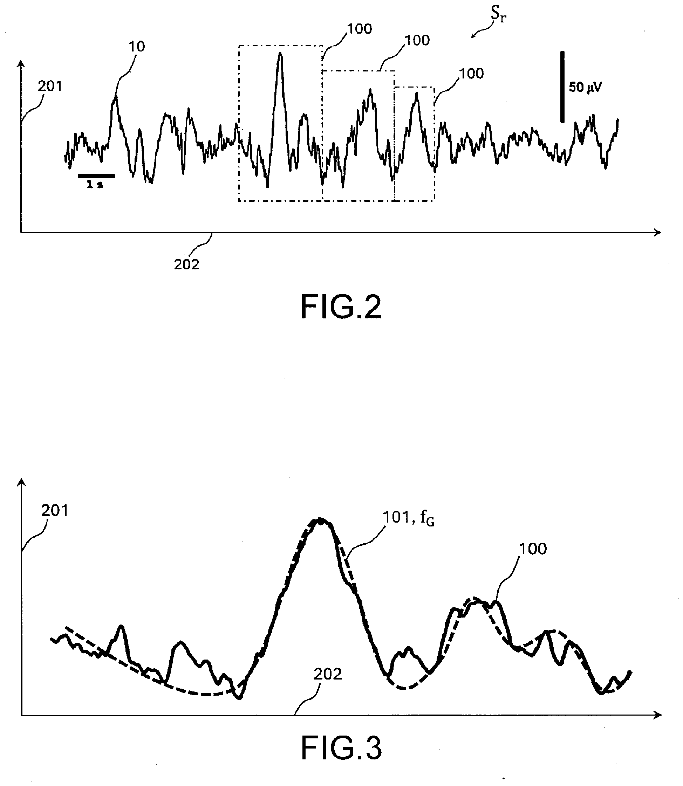

Figure 2 illustrates an example of a physiological signal 10 acquired. The physiological signal 10 is plotted in a graph, where the vertical axis 201 represents the amplitude of the signal, and the horizontal axis 202 represents the elapsed time. The amplitude of the physiological signal 10 can be for example expressed in microvolts ($\mu$V), when the physiological signal 10 is a brain wave. The time can be expressed in seconds (s) or milliseconds (ms). This physiological signal 10 comprises a succession of raw patterns 100, wherein each pattern 100 is defined in a dashed square according to the example illustrated in Figure 2. This succession of patterns 100 forms the raw sequence $S_r$.

Figure 3 represents a part of the physiological signal 10 of Figure 2 (the part indicated by the first dashed rectangle starting from the left in Figure 2). Figure 3 shows a raw pattern 100 which will be taken as an example in order to describe the invention hereinafter.

As illustrated in Figure 1, the method further comprises a generation step E2 of a sensory signal 30 associated with the raw sequence $S_r$. In the box E2 of the diagram of Figure 1, "GEN 30" means "generation of a sensory signal 30".

The generation step E2 can comprise one or several sub-steps, as shown in Figure 1. The generation step E2 comprises a transformation E21 of the raw sequence $S_r$ into a modulated sequence $S_m$ of modulated patterns 101. In other words, in the transformation step E21, each raw pattern 100 is processed, or in other words 'fitted', to obtain a corresponding modulated pattern 101. In the box E21 of the diagram of Figure 1, "TRANS $S_r$ - $S_m$" means "transformation of the raw sequence $S_r$ into a modulated sequence $S_m$". The succession of processed patterns, or modulated patterns 101, forms a modulated sequence Sm.

In the transformation E21 step, each raw pattern 100 is fitted with a sum of at least one Gaussian function $f_G$, expressed as follows:

$$f_G = \sum_{i=1}^{N} A_i exp\left(\frac{-(t - \mu_i)^2}{2\sigma_i^2}\right)$$

where the parameter:

- i is the $i^{th}$ Gaussian function in the sum,
- $N$ is the total number of Gaussian functions in the sum,
- $A_i$ is the amplitude of the $i^{th}$ Gaussian function in the sum,
- t is the time,
- $\mu_i$ is the mean or central value of the $i^{th}$ Gaussian function or distribution, indicating the location of the peak or center of the $i^{th}$ Gaussian function along the horizontal axis, and
- $\sigma_i$ is the standard deviation of the $i^{th}$ Gaussian distribution. It characterizes the spread or width of the distribution.

Each of these parameters can be approximated using numerical fitting techniques. The amplitude of the Gaussian function can be proportional to the amplitude of the raw pattern 100, and the standard deviation can be proportional to the width of the raw pattern 100.

The transformation step can include an initial peak detection, in which an approximate location of each significant peak corresponding to a maximal amplitude of a raw pattern is identified. This can be done by simple thresholding, in which points where the signal exceeds a certain threshold are detected, or it can be done using more advanced techniques. By identifying the peaks, it is possible to emphasize significant parts of the physiological signal, and to ignore noise and other irrelevant parts of the physiological signal, leading to a cleaner processing.

The transformation step can also include constraining the width of the Gaussian function. An upper and lower bounds for the standard deviation can be set to control the width of the Gaussian. These bounds can be chosen in accordance with the nature of the physiological signal. A minimal width of 2 ms can be set, which covers a large set of physiological signals. For instance, waves detected in deep sleep, are characterized by frequencies ranging from 1 Hz to 4 Hz. Consequently, the processing of a physiological signal acquired in a state of deep sleep, can include the setting of a width of the Gaussian function between 20 ms and 50 ms. These boundaries can help preventing the fitting of noise patterns.

Consequently, each raw pattern can be fitted by an initial sum of at least one Gaussian function whose parameters are then optimized to obtain a modulated pattern that spouses the raw pattern. The purpose of the optimization process is to minimize the error between the fitted pattern, i.e. modulated pattern, and the raw pattern. This process often involves an iterative algorithm with a feedback loop, to refine the parameters of the sum of Gaussian functions, until the error converges to a minimum value. In each iteration of the algorithm, the parameters of the sum of Gaussian functions can be updated based on the error calculated in the previous step, or an additional Gaussian function can be added to the sum of the Gaussian functions. The user can set a minimum acceptable error or a convergence criterion, and the optimization can stop until this minimum error is reached. Below this error threshold, each of the raw patterns is fitted with an optimized sum of Gaussian functions that satisfies the user. The minimum acceptable error depends on the noise level in the physiological signal 10. According to an example, the R-squared can be used to determine whether the Gaussian fitting is accurate. The R-squared shows how well the data fit a regression model (the goodness of fit). An R-squared value higher than 0.7 can be set for example, to ensure a good fitting with the Gaussian function.

As illustrated in Figure 3, the raw pattern 100 is processed into a modulated pattern 101, or envelope, represented with a dashed line. This modulated pattern 101 is obtained by fitting the raw pattern 100 with the sum of Gaussian functions $f_G$, expressed hereabove. This sum of Gaussian functions fc allows the modulation of the raw pattern 101 in a faithful manner, meaning that the envelope follows closely the variation of the raw pattern 101 in time. The obtained modulated pattern 101 is thus an accurate replication of the raw pattern 100. The sum of Gaussian functions forms thereby the envelope of the sensory signal or stimuli such as acoustic stimuli (e.g. music, sounds) or visual stimuli (e.g. light, images), *etc.* Such a tailored stimuli can engage various cognitive processes in the organism's brain, evoking emotional responses for example, or activating the brain's reward system, inducing sleep, or enhancing attention.

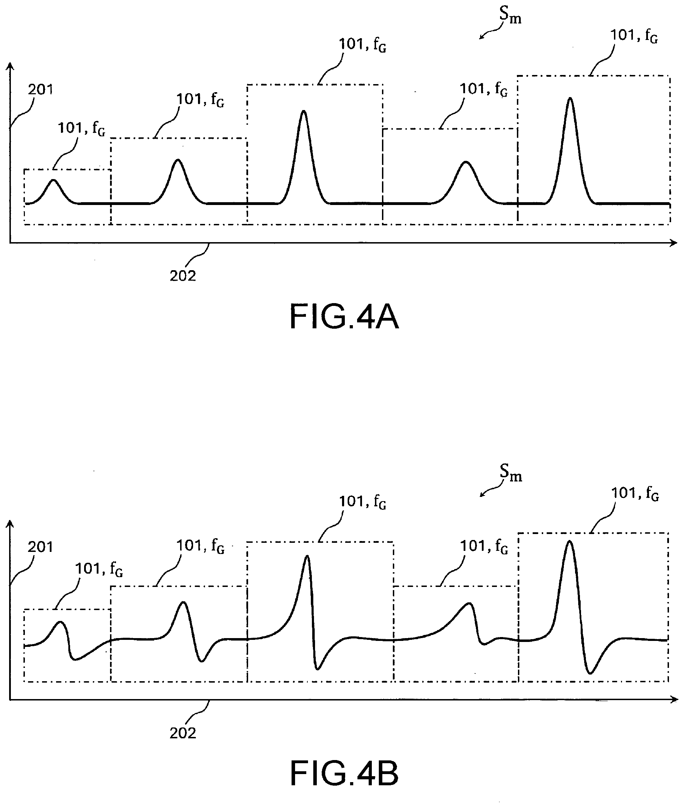

By modulating each of the raw patterns 100 with a sum of Gaussian functions $f_G$, a modulated sequence $S_m$ comprising a succession of modulated patterns 101 can therefore be obtained, as illustrated in Figures 4A and 4B.

As illustrated in Figure 4A, according to an embodiment, each raw pattern 101 can be modulated by a single Gaussian function fc. In this embodiment, a single Gaussian function assumes that the raw pattern 100 follows a symmetric, bell-shaped distribution around a central value, or peak. If the raw pattern 101 contains multiple peaks, asymmetries or other complex features, a sum of multiple Gaussian functions $f_G$ can advantageously describe more accurately the underlying features of the raw pattern.

As illustrated in Figures 3 and 4B, according to another embodiment, each raw pattern 101 can be modulated by a sum of two or more Gaussian functions $f_G$, which allows to fit closely the fine structure of the raw pattern 100.

In both embodiments illustrated in Figures 4A and 4B, the modulated sequence $S_m$ comprises multiple modulated patterns 101. Advantageously, the transformation E21 step can be adapted in a manner that the sums of Gaussian functions $f_G$ of two successive modulated patterns 101 overlap. This is facilitated by the use of a continuous mathematical function throughout the processing, such as the Gaussian function. In such a manner, the modulated sequence $S_m$, represents a continuous flow of modulated patterns 101, forming a continuous envelope. Such a continuous envelope with no interruptions or sudden changes, allows the generation of a smooth sensory signal 30, whose variations adhere closely to the successive patterns 100 of the physiological signal 10.

The resulting modulated sequence $S_m$ can then be used during the generation E2 step, to modulate a sequence of pre-recorded signals or samples.

According to a variant, the sensorial signal 30 can be generated concurrently with the acquisition E1 of the physiological signal 10. As soon as a first raw pattern 100 is present in a part of the physiological signal 10 being acquired, a sequence $S_r$ of raw patterns is initiated, and the carrying out of the step E2 is triggered. A first modulated pattern 110 is then obtained by numerically fitting the raw pattern 100, a modulated sequence $S_m$ is then initiated in concert with the raw sequence $S_r$. The generation of a sensory signal 30 is then initiated, by modulating a first pre-recorded signal to the first modulated pattern 101. As soon as a second raw pattern 100 is acquired, following the acquisition of the first raw pattern, this second raw pattern 100 is added to the raw sequence $S_r$, a second modulated pattern 101 is added to the modulated sequence $S_m$, and a second pre-recorded signal is modulated to the second modulated pattern 101. This loop of steps or sub-steps is continuously repeated as long as raw patterns 100 are acquired in the physiological signal 10. Consequently, the presence of several raw patterns 100 in the physiological signal 10 is reflected by successive modulations of pre-recorded signals associated with these different patterns.

The step of generation E2 of the sensory signal 30, including the sub-step of transformation E21 can thus be carried out in real time with respect to the acquisition E1 of the physiological signal 10.

According to another variant, the physiological signal 10 can be acquired in a prior or independent step E1. The following steps can also be carried out separately. For instance, after the acquisition E1 of a complete raw sequence $S_r$ in the physiological signal 10, the raw sequence $S_r$ can then be transformed into a modulated

sequence $S_m$ in a single transformation step E21. The continuous envelope formed by the modulated sequence $S_m$ can then be modulated to the sequence of pre-recorded signals during the generation E2 of the sensory signal 30.

Consequently, the transduction method is carried out by passing through an intermediate recording of the physiological signal 10, i.e. the raw sequence $S_r$, and/or the modulated sequence $S_m$ in a memory of computer data or of a digital or electronic or electric or analogue signal (for example a sound file) allowing the subsequent generation of the sensory signal 30. Additionally, the pre-recorded signals can be stored in the memory of the same computer.

The generated sensory signal 30 can comprise several components, in addition to the envelope produced by the sums of Gaussian functions. These additional components are described hereafter in reference to figures 1, 5 and 6.

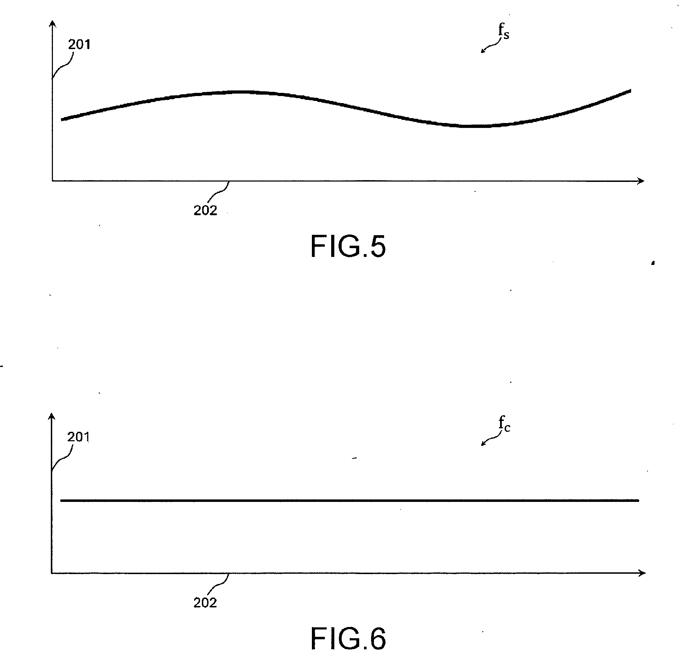

As illustrated in Figures 1 and 5, the generation E2 of the sensory signal 30, can comprise a first sub-generation E22 of a "slow" periodic function $f_s$. In the box E22 of the diagram of Figure 1, "Sub-GEN $f_s$" means "sub-generation of a slow periodic function $f_s$".

This periodic function $f_s$ is labeled as "slow" relatively to an average periodicity of the physiological signal 10. In fact, the physiological signal 10 can comprise multiple raw patterns 100 that can be separated in time, giving rise to an average periodicity. The raw sequence $S_r$ and the resulting modulated sequence $S_m$ can therefore adopt the average periodicity of the raw patterns in the physiological signal 10. The sub-generation E22 of the slow periodic function $f_s$ is advantageously configured, so that the periodicity of the slow periodic function $f_s$ can be 5 to 80 times greater than the average periodicity of the modulated sequence $S_m$.

in an advantageous embodiment, the slow periodic function $f_s$ is generated independently from the raw sequence $S_r$. Consequently, the periodic function $f_s$ can have an amplitude $A_s$ different than the amplitude of the physiological signal 10. Figure 5 illustrates an example of a slow periodic function $f_s$ that can be added to the modulated sequence $S_m$ of Figures 4A or 4B.

As illustrated in Figures 1 and 6, the generation E2 of the sensory signal 30, can further comprise a second sub-generation E23 of a constant function $f_c$. in the box E23 of the diagram of Figure 1, "Sub-GEN $f_c$" means "sub-generation of a constant function $f_c$". The constant function $f_c$ presents a constant amplitude $A_c$ that is greater than zero. Figure 6 illustrates an example of a constant function $f_c$ that can be added to the modulated sequence $S_m$ of Figures 4A or 4B. The constant non-zero amplitude $A_c$ of the constant function helps avoiding interruptions in the generated sensory signal 30.

The generated sensory signal 30 can thus comprise three different components, namely, a first component resulting from the sums of Gaussian functions $f_G$, a second component resulting from the slow periodic function $f_s$ and a third component resulting from the constant function $f_c$. The maximal amplitude of the sensory signal 30 is denoted as $A_{30}$. Advantageously, the first component resulting from the sums of Gaussian functions $f_G$, does not produce the total maximal amplitude $A_{30}$ of the sensory signal. Preferably, the maximal amplitude $A_G$ of the modulated sequence $S_m$, i.e. of the component resulting from the sums of Gaussian functions $f_G$, represents a fraction of 20% to 40% of the maximal amplitude $A_{30}$. The other fraction of the maximal amplitude $A_{30}$ can be contributed by the other two components. For instance, the maximal amplitude $A_s$ of the slow periodic function $f_s$ can represent a fraction of 20% to 40% of the maximal amplitude $A_{30}$, and the constant amplitude $A_c$ of the constant function $f_c$ can represent a fraction 20% to 40% of the maximal amplitude $A_{30}$.

All the above-mentioned steps can be numerically carried out, given that the processing of the signal is carried out by using mathematical calculations, and that the pre-recorded signals can be provided as numerical data base. The method can therefore be fully automated, from the acquisition E1 of the physiological signal 10 to the generation E2 of the sensory signal 30, through the modulation of the pre-recorded signals. This simplifies the application of the present method.

As an example, the physiological signal can be representative, for example, of a human brain activity, or an animal brain activity. An example of a physiological signal coming from a human brain activity is represented in Figure 1. The physiological signal 10 of Figure 1 can be obtained with the aid of an electroencephalogram (EEG) recording the brain activity of a human being in a state of deep sleep. Alternatively, dry electrodes can be used, or wet electrodes with or without conductive gel or paste.

The physiological signal 10 can correspond to the brain activity of a wakeful human being. In another embodiment, it can be representative of a state of deep sleep and corresponds to a delta wave.

These different cognitive states of deep sleep or of wakefulness, are characterized by the presence of waves which oscillate at specific frequencies. Typically, in a state of deep sleep, the brain can generate waves, called delta waves, having a frequency comprised between 0.3 and 4 Hz. In a state of wakefulness, the brain can generate waves, called beta waves, typically having a frequency greater than 14 Hz.

The present invention is not limited to the acquisition or to the processing of the delta or beta waves and can be based on an acquisition of a physiological signal representative of a physiological activity, for example cerebral, cardiac, respiratory, ocular or else muscular - of any organism - for example an animal or a human being. This organism, in particular when the latter is an animal or a human being, can be placed in any cognitive state - for example in a state of deep or paradoxical sleep or of wakefulness or else of relaxation.

The sensory signal 30 is preferably an acoustic signal or a wave but can alternatively or additionally be a signal or a wave of any other kind (visual, tactile, olfactory etc.) provided that it is perceptible to said organism.

In the embodiment in which the sensory signal 30 is an acoustic signal, the present method allows the generation of a sound that is continuous without interruption or moments of silence, due to the generation of a constant function $f_c$. Moreover, the generated sound is configured to be relaxing and soothing. In fact, the modulation of the sensory signal to Gaussian functions, replicates faithfully the patterns in the brain signal. Consequently, the generated sound helps the organism to relax given that the sound reflects his brain activity. In addition to the faithful replication of the patterns, adding a slow periodic function $f_s$ can further enhance the relaxation of the organism. The periodicity of the slow periodic function $f_s$ can be comprised between 10 seconds and 20 seconds. Such a periodicity is slower than the average breathing period of a human being. This has a relaxing auditory effect.

Moreover, he pre-recorded signals can be for example "music samples". These samples can be natural sounds including water flowing sound, waves sound, rain falling sound, whales sound, *etc.* These sounds can be inherently relaxing due to their ability to trigger positive emotions, mask external noise, reduce stress levels, and evoke feelings of safety and comfort associated with natural environments. By listening to these sounds, especially when they are modulated by the brain activity, the generated sound can improve sleep quality and overall well-being.

A physio-sensory transduction device capable of implementing the method detailed earlier is now described.

The physio-sensory transduction device comprises an acquisition means, arranged and/or programmed to implement the acquisition step E1, consisting of acquiring a physiological signal 10 from an organism, comprising the raw sequence $S_r$ of raw patterns 100. Such an acquisition step E1 can be carried out with the aid of a physio-sensory transduction device.

The physio-sensory transduction device further comprises a generation system arranged and/or programmed to carry out the generation step E2 of a sensory signal 30 associated with the raw sequence $S_r$, as well as the sub-steps E22 and E23. The generation system can comprise several means, such as a first modulation means that is arranged and/or programmed to transform the raw sequence $S_r$ into a modulated sequence $S_m$ of modulated patterns 101. This modulation means is therefore programmed to carry out a fitting procedure using a sum of Gaussian functions fc, to fit the raw patterns 100. The generation system can further comprise two sub-generation means, each of them being arranged and/or programmed to generate, respectively, the slow periodic function $f_s$ and the constant function $f_c$.

The generation system can further comprise a data base of pre-recorded signals, stored in a memory, and a second modulation means arranged and/or programmed to modulate the pre-recorded signals to the modulated sequence $S_m$ during the generation step E2 of the sensory signal 30.

In an embodiment, the generation system can be a computing device comprising a processor and a memory, and in which the first modulation means, the two sub-generation means, the data base of pre-recorded signals and the second modulation means are all embedded. This computing device can thus be connected to acquisition means, in order to transfer directly the physiological signal 10 to the computing device, where all the following steps and sub-steps can be carried out, in real time or in a deferred way. Consequently, the method can be fully automated via the computing device.

In the embodiment where the steps are carried out independently from each other, the memory of the computing device can also be used to store the physiological signal 10, and/or the raw sequence $S_r$, and/or the modulated sequence $S_m$, in a way that these stored data can be used independently and at any time to carry out any of the associated steps of the method.

The first modulation means and the two sub-generation means can comprise for example algorithms embedded in the computing device. These algorithms are generally used in signal processing and data analysis. They can be implemented in software applications such as signal processing libraries, data analysis software packages, custom-written code, or artificial intelligence. They may include instructions stored on one or more non-transitory medium, and that can be executed by one or more processors.

Of course, the invention is not limited to the examples which have just been described and numerous adjustments may be made to these examples without exceeding the scope of the invention.

For example, the method can comprise a classification step making it possible to select patterns representative of a particular cognitive state. Such a classification step can be useful, in particular in a situation in which the chosen pattern selection criteria are capable of selecting patterns representative of different cognitive states between which it is desired to make a distinction. This could be the case after acquisition of a physiological signal representing a brain activity over a prolonged duration, such as one night.

Furthermore, the different characteristics, forms, variants and embodiments of the invention can be combined together in various combinations, to the extent that they are not incompatible or mutually exclusive.

The physiological signal can be representative of a brain activity of the organism (alpha, beta, delta waves *etc.)* or of a cardiac or respiratory or ocular or muscular activity of the organism of an animal or a human being. The physiological signal is not acquired from this animal or human being in an invasive manner, for example not by means of an intracerebral probe.

As explained above, the sensory signal can be an acoustic or visual or tactile or olfactory or gustatory signal. The sensory signal is preferably received (for example heard) by the same animal or human being from which or from whom the physiological signal has originated. The term "personalized" sensory signal is then used.

This sensory signal can be replayed in a personalized manner to the same individual who produced the sensory signal, or more generally to another user.

Of course, the different characteristics, forms, variants and embodiments of the invention can be combined together in various combinations.

**Claims**

1. Physio-sensory transduction method comprising:

   - an acquisition (E1) of a physiological signal (10) of an organism, said physiological signal (10) comprising a raw sequence ($S_r$) of at least one raw pattern (100), and
   - a generation (E2) of a sensory signal (30) associated with the raw sequence ($S_r$),

   the method being **characterized in that** the generation (E2) of the sensory signal (30) comprises:

   ○ a transformation (E21) of the raw sequence ($S_r$) into a modulated sequence ($S_m$), said modulated sequence ($S_m$) comprising at least one modulated pattern (101), wherein each modulated pattern (101) is obtained by fitting the raw pattern (100) with a sum of at least one Gaussian function ($f_G$), said sum of at least one Gaussian function (fc) forming an envelope to each raw pattern (100).

2. Method according to claim 1, wherein the modulated sequence ($S_m$) comprises at least two successive modulated patterns (101), and wherein the transformation (E21) of the raw sequence ($S_r$) is configured in a way that the sums of at least one Gaussian function ($f_G$), associated with the at least two successive modulated patterns (101), overlap.

3. Method according to any one of claims 1 and 2, wherein the generation (E2) of the sensory signal (30) further comprises:

   ○ a first sub-generation (E22) of a periodic function ($f_s$) that is added to the modulated sequence ($S_m$), said periodic function ($f_s$) having a periodicity comprised between 10 seconds and 20 seconds.

4. Method according to claims 3, wherein the periodic function ($f_s$) has a maximal amplitude $A_s$ that represents a first fraction of a maximal amplitude $A_{30}$ of the generated sensory signal (30), said first fraction being comprised between 20% and 40%.

5. Method according to any one of claims 1 to 4, wherein the generation (E2) of the sensory signal (30) further comprises:

   ○ a second sub-generation (E23) of a constant function ($f_c$) that is added to the modulated sequence ($S_m$), said constant function ($f_c$) having a constant amplitude $A_c$ greater than zero.

6. Method according to the preceding claim, wherein the constant amplitude Ac of the constant function (fc) represents a second fraction of a maximal amplitude A30 of the generated sensory signal (30), said second fraction being comprised between 20% and 40%.

7. Method according to any one of claims 1 to 6, wherein the modulated sequence Sm has a maximal amplitude AG that represents a third fraction of a maximal amplitude A30 of the generated sensory signal (30), said third fraction being comprised between 20% and 40%.

8. Method according to any one of claims 1 to 7, wherein each sum of at least one Gaussian function (fG) comprises a single Gaussian function (fG).

9. Method according to any one of claims 1 to 7, wherein each sum of at least one Gaussian function ($f_G$) comprises at least two Gaussian functions ($f_G$).

10. Method according to any one of claims 1 to 9, wherein the generation (E2) of the sensory signal (30) is carried out using a sequence of pre-recorded signals modulated by at least the modulated sequence ($S_m$).

11. Method according to any one of claims 1 to 10, wherein the generation (E2) of the sensory signal (30) is carried out in a deferred manner with respect to the acquisition (E1) of the physiological signal (10).

12. Method according to any one of claims 1 to 10, wherein the generation (E2) of the sensory signal (30) is carried out in real time with respect to the acquisition (E1) of the physiological signal (10).

13. Method according to any one of claims 1 to 12, wherein:

    - the organism is a human being or an animal,
    - the physiological signal (10) is representative of a brain activity of said human being or said animal, and

- the sensory signal (30) is an acoustic signal.

**14.** Physio-sensory transduction device comprising:

- an acquisition means configured to acquire a physiological signal (10) from an organism, said physiological signal (10) comprising a raw sequence ($S_r$) of at least one raw pattern (100), and
- a generation system configured to generate a sensory signal (30) associated with the raw sequence ($S_r$),

the device being **characterized in that** the generation system is further configured to transform the raw sequence ($S_r$) into a modulated sequence ($S_m$), said modulated sequence ($S_m$) comprising at least one modulated pattern (101), wherein each modulated pattern (101) is obtained by fitting the raw pattern (100) with a sum of at least one Gaussian function ($f_G$).

ACQ **10** — E1

GEN **30** — E2

TRANS $S_r - S_m$ — E21

Sub-GEN $f_s$ — E22

Sub-GEN $f_c$ — E23

FIG.1

FIG.2

FIG.3

FIG.4A

FIG.4B

FIG.5

FIG.6

Europäisches
Patentamt
European
Patent Office
Office européen
des brevets

# EUROPEAN SEARCH REPORT

**Application Number**

EP 24 31 5354

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROH SEONG-EUN ET AL: "A new action potential classifier using 3-Gaussian model fitting", ARXIV,, vol. 71, no. 16-18, 22 April 2008 (2008-04-22), pages 3631-3634, XP025680879, DOI: 10.1016/J.NEUCOM.2008.04.002 [retrieved on 2008-04-22] | 1-12,14 | INV. A61B5/375 A61M21/02 G06F3/01 |
| A | * sections 2. and 3.; figure 2(a) * | 13 | |
| A | DESTEXHE ALAIN ET AL: "A method to convert neural signals into sound sequences", THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 151, no. 6, 2 June 2022 (2022-06-02), pages 3685-3689, XP012266142, ISSN: 0001-4966, DOI: 10.1121/10.0011549 [retrieved on 2022-06-02] * sections II. and III.; figures 1-4 * | 1-14 | |
| | | | **TECHNICAL FIELDS SEARCHED (IPC)** |
| | | | A61B A61M G06F |
| A | US 2020/138322 A1 (DESTEXHE ALAIN [FR] ET AL) 7 May 2020 (2020-05-07) * paragraph [0170] - paragraph [0225]; figures 1-5 * | 1-14 | |

The present search report has been drawn up for all claims

| Place of search | Date of completion of the search | Examiner |
|---|---|---|
| The Hague | 12 November 2024 | Mecking, Nikolai |

EPO FORM 1503 03.82 (P04C01)

## ANNEX TO THE EUROPEAN SEARCH REPORT
## ON EUROPEAN PATENT APPLICATION NO.

EP 24 31 5354

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2024

| Patent document cited in search report | Publication date | Patent family member(s) | | Publication date |
|---|---|---|---|---|
| US 2020138322 A1 | 07-05-2020 | CA | 3058976 A1 | 01-11-2018 |
| | | EP | 3614906 A1 | 04-03-2020 |
| | | FR | 3065366 A1 | 26-10-2018 |
| | | JP | 7169990 B2 | 11-11-2022 |
| | | JP | 2020517372 A | 18-06-2020 |
| | | US | 2020138322 A1 | 07-05-2020 |
| | | WO | 2018197155 A1 | 01-11-2018 |

EPO FORM P0459

For more details about this annex : see Official Journal of the European Patent Office, No. 12/82

**EP 4 684 731 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 3065366 B1